# EUROPEAN PATENT APPLICATION

(11) **EP 2 238 930 A2**
(43) Date of publication of application: **13.10.2010**
(21) Application number: 10250731.6
(22) Date of filing: 08.04.2010
(51) Int. Cl.: A61B 17/34

(54) **Surgical access device with one time seal**

(30) Priority: 08.04.2009 US 420232
(71) Applicant: Ethicon Endo-Surgery, Inc., Cincinnati, Ohio 45242-2839 (US)
(72) Inventor: Widenhouse, Christopher W., Clarksville Ohio 45113 (US); Schmid, Katherine J., Cincinnati, Ohio 45241 (US); Nguyen, Anthony T., West Chester Ohio 45069 (US); Herrera-Davis, Denzel Z., Cincinnati, Ohio 45236 (US); Minnelli, Patrick J., Harrison Ohio 4030 (US); Shelton, Frederick E. IV, Hillsboro Ohio 45133 (US)
(74) Representative: Tunstall, Christopher Stephen

(57) **Abstract**

Various methods and devices are provided for sealing a working channel of a surgical access device using a non-resealable membrane. The non-resealable membrane seals the working channel of the device before use and is punctured when a surgical instrument is inserted into the device such that it will no longer provide a seal in the working channel.

## Description

### FIELD OF THE INVENTION

The present invention relates to methods and devices for providing surgical access into a body cavity.

### BACKGROUND OF THE INVENTION

Abdominal laparoscopic surgery gained popularity in the late 1980's, when benefits of laparoscopic removal of the gallbladder over traditional (open) operation became evident. Reduced postoperative recovery time, markedly decreased post-operative pain and wound infection, and improved cosmetic outcome are well established benefits of laparoscopic surgery, derived mainly from the ability of laparoscopic surgeons to perform an operation utilizing smaller incisions of the body cavity wall.

Many laparoscopic procedures involve insufflation of the abdominal cavity with CO2 gas to a pressure of around 15 mm Hg to increase interior space for a surgical procedure. The abdominal wall is pierced and a surgical access device, such as a straight tubular cannula or trocar sleeve, is then inserted into the abdominal cavity. The surgical access device must be sealed to the outside in order to achieve and maintain insufflation once the surgical access device is positioned within tissue. Thus, various sealing elements are used within the surgical access device to seal its working channel both before and after a surgical instrument is inserted. One type of sealing element called a channel seal acts to seal the working channel when there is no surgical instrument inserted through the surgical access device. Channel seals can have relatively large vertical profiles and can use valuable space within the surgical access device, as well as can limit angular movement of a surgical instrument within the body. In many procedures, however, channel seals are only needed during initial insufflation of the abdomen before a surgical instrument is inserted and will no longer be utilized after a surgical instrument is inserted.

Thus, it would be desirable to have a small profile sealing element that seals the working channel of a surgical access device before a surgical instrument is inserted and is not required to provide further sealing of the.working channel after a surgical instrument is inserted.

### SUMMARY OF THE INVENTION

The present invention generally provides methods and devices for surgically accessing an interior of a patient's body. In one embodiment, a surgical access device is provided and can include a housing having an access tube extending distally therefrom. The housing can have at least one port formed therein that defines a working channel in communication with a working channel of the access tube. A non-resealable membrane can extend across the working channel in at least one of the at least one port and the access tube to provide a seal across the working channel until a surgical instrument is punctured through the membrane.

In an exemplary embodiment, the port can include at least one sealing element disposed therein and configured to form a seal around an instrument inserted therethrough. The non-resealable membrane can be positioned anywhere in the housing, the access tube, and/or the port. For example, the non-resealable membrane can be positioned proximal to the sealing element and/or distal to the sealing element. The non-resealable membrane can also be positioned at an angle greater than, equal to, or less than 90 degrees, with respect to a central longitudinal axis of the port. In some embodiments, the non-resealable membrane can include a plurality of perforations formed therein and configured to provide a predefined puncture location to facilitate puncturing of the membrane by a surgical instrument. The non-resealable membrane can also have many shapes and thicknesses, and in one embodiment, the non-resealable membrane is substantially flat and extends across the port in a direction perpendicular to a central longitudinal axis of the port. In another embodiment, the non-resealable membrane can be positioned proximal to the sealing element and can be configured to protect the sealing element when a surgical instrument is inserted therethrough.

The access tube can have many configurations. In one embodiment, the access tube can include at least one flexible elongate sealing element extending distally therefrom and configured to form a seal around a surgical instrument inserted therethrough. In another embodiment, the access tube can be a flexible retractor and/or a rigid cannula. In some embodiments, a diameter of the flexible retractor can be mechanically adjustable for insertion into and removal from an opening in a body. The non-resealable membrane can also include a support member that is configured to provide support to at least one predefined region of the non-resealable membrane when a tool is inserted through the predefined region. In one embodiment, the housing can include a plurality of ports, each port having a non-resealable membrane extending across a working channel of the port.

In other aspects, methods of providing surgical access through tissue and into a body are also provided and can include inserting a distal portion of a surgical access device through an opening in tissue to position a distal end of the surgical access device in a body cavity, and providing an insufflation gas through a working channel of the surgical access device to insufflate the body cavity. A membrane can extend across the working channel to form a seal in the working channel that prevents escape of the insufflation gas from the body cavity. The method can further include inserting a surgical instrument through the working channel of the surgical access device, thereby puncturing the membrane such that the membrane is incapable of sealing the working channel when the surgical instrument is removed.

In some embodiments, inserting a surgical instrument through the working channel of the surgical access device can include inserting the surgical instrument through at least one sealing element that forms a seal around the surgical instrument. In one aspect, the membrane can protect a proximal surface of the sealing element from being damaged by the surgical instrument. Puncturing the membrane can include inserting the surgical instrument through predefined perforations formed in the membrane. In addition, inserting a distal portion of a surgical access device through an opening in tissue can further include mechanically adjusting a diameter of the distal portion.

In another exemplary embodiment, a surgical access device is provided and can include an access tube having an opening extending therethrough for forming a pathway through tissue into a body cavity and a housing coupled to the access tube. The housing or access tube can have at least one sealing member therein. The sealing member can have a variety of configurations, and in one embodiment can be a plurality of sealing membranes positioned one on top of another with each sealing membrane having a plurality of slits formed therethrough. The sealing membranes can be oriented such that the slits in each sealing membrane are substantially offset from one another to form a seal around a surgical instrument inserted through the sealing member. The slits can be oriented to form a seal 360 degrees around a surgical instrument. In some embodiments, the slits in each sealing membrane can include parallel slits positioned across a diameter of each sealing membrane. The sealing membranes can be formed of a substantially flexible material such that the slits in each membrane are configured to flex open and closed to receive a surgical instrument therethrough. There can be any number of membranes in the plurality of membranes, for example, at least ten membranes.

In another embodiment, the sealing member can be a first sealing member and a second sealing member that are separated by a gap. A plurality of pellets can be disposed in the gap and configured to prevent the escape of insufflation gas. The surgical access device can also include an insufflation port in communication with the gap between the first sealing member and the second sealing member. In some embodiments, the second sealing member can include at least five membranes.

In other aspects, a method of providing surgical access within a body is provided and can include inserting a surgical instrument through multiple layers of a first sealing element such that slits formed in each layer of the sealing element form a seal around the surgical instrument. The slits formed in each layer of the sealing element can flex open and closed to receive and seal around the surgical instrument inserted therethrough. The method can also include inserting a surgical instrument through multiple layers of a second sealing element such that slits formed in each layer of the second sealing element form a seal around the surgical instrument. In addition, a predetermined gas pressure can be provided within a space between the first and second sealing elements.

The following is a non-exhaustive list of embodiments which are or may be claimed:
1. A surgical access device, comprising:
   a housing having an access tube extending distally therefrom, the housing having at least one port formed therein and defining a working channel in communication with a working channel of the access tube; and
   a non-resealable membrane extending across the working channel in at least one of the at least one port and the access tube, the membrane providing a seal across the working channel until a surgical instrument is punctured through the membrane.
2. The surgical access device of embodiment 1, wherein the at least one port includes at least one sealing element disposed therein configured to form a seal around an instrument inserted therethrough.
3. The surgical access device of embodiment 2, wherein the non-resealable membrane is positioned proximal to the at least one sealing element.
4. The surgical access device of embodiment 2, wherein the non-resealable membrane is positioned distal to the at least one sealing element.
5. The surgical access device of embodiment 1, wherein the non-resealable membrane includes a plurality of perforations formed therein configured to provide a predefined puncture location to facilitate puncturing of the membrane by a surgical instrument.
6. The surgical access device of embodiment I, wherein the non-resealable membrane is substantially flat and extends across the at least one port in a direction perpendicular to a central longitudinal axis of the at least one port.
7. The surgical access device of embodiment 2, wherein the non-resealable membrane is positioned proximal to the at least one sealing element and is configured to protect the sealing element when a surgical instrument is inserted therethrough.
8. The surgical access device of embodiment 1, wherein the non-resealable membrane is positioned at an angle less than 90 degrees with respect to a central longitudinal axis of the port.
9. The surgical access device of embodiment 1, wherein the access tube includes at least one flexible elongate sealing element extending distally therefrom and configured to form a seal around a surgical instrument inserted therethrough.
10. The surgical access device of embodiment 1, wherein the access tube comprises a flexible retractor.
11. The surgical access device of embodiment 1, wherein the access tube comprises a rigid cannula.
12. The surgical access device of embodiment 1, wherein the non-resealable membrane includes a support member that is configured to provide support to at least one predefined region of the non-resealable membrane when a tool is inserted through the predefined region.
13. The surgical access device of embodiment 1, wherein the housing includes a plurality of ports, each port having a non-resealable membrane extending across a working channel of the port.
14. A method of providing surgical access within a body, comprising:
   inserting a distal portion of a surgical access device through an opening in tissue to position a distal end of the surgical access device in a body cavity;
   providing an insufflation gas through a working channel of the surgical access device to insufflate the body cavity, a membrane extending across the working channel forming a seal in the working channel that prevents escape of the insufflation gas from the body cavity; and
   inserting a surgical instrument through the working channel of the surgical access device, the instrument puncturing the membrane such that the membrane is incapable of sealing the working channel when the surgical instrument is removed.
15. The method of embodiment 14, wherein inserting a surgical instrument through the working channel of the surgical access device includes inserting the surgical instrument through at least one sealing element that forms a seal around the surgical instrument.
16. The method of embodiment 15, wherein the membrane protects a proximal surface of the at least one sealing element from being damaged by the surgical instrument.
17. The method of embodiment 14, wherein puncturing the membrane comprises inserting the surgical instrument through predefined perforations formed in the membrane.
18. The method of embodiment 14, wherein inserting a distal portion of a surgical access device through an opening in tissue further comprises mechanically adjusting a diameter of the distal portion.
19. A surgical access device, comprising:
   an access tube having an opening extending therethrough for forming a pathway through tissue into a body cavity; and
   a housing coupled to the access tube and having at least one sealing member therein, the at least one sealing member having a plurality of sealing membranes positioned one on top of another with each sealing membrane having a plurality of slits formed therethrough, the plurality of sealing membranes being oriented such that the plurality of slits in each sealing membrane are substantially offset from one another to form a seal around a surgical instrument inserted through the at least one sealing member.
20. The surgical access device of embodiment 19, wherein the plurality of slits are oriented to form a seal 360 degrees around a surgical instrument.
21. The surgical access device of embodiment 19, wherein the plurality of slits in each sealing membrane comprise parallel slits positioned across a diameter of each sealing membrane.
22. The surgical access device of embodiment 19, wherein the plurality of sealing membranes are formed of a substantially flexible material such that the plurality of slits in each membrane are configured to flex open and closed to receive a surgical instrument therethrough.
23. The surgical access device of embodiment 19, wherein the plurality of membranes comprise at least ten membranes.
24. The surgical access device of embodiment 19, wherein the at least one sealing member comprises a first sealing member and a second sealing member, the first and second sealing members being separated by a gap.
25. The surgical access device of embodiment 24, further comprising a plurality of pellets disposed in the gap and configured to prevent the escape of insufflation gas.
26. The surgical access device of embodiment 24, further comprising an insufflation port in communication with the gap between the first sealing member and the second sealing member.
27. The surgical access device of embodiment 24, wherein the plurality of membranes of the second sealing member comprise at least five membranes.
28. A method of providing surgical access within a body, comprising:
   inserting a surgical instrument through multiple layers of a first sealing element such that slits formed in each layer of the sealing element form a seal around the surgical instrument, wherein the slits formed in each layer of the sealing element flex open and closed to receive and seal around the surgical instrument inserted therethrough.
29. The method of embodiment 28, further comprising inserting a surgical instrument through multiple layers of a second sealing element such that slits formed in each layer of the second sealing element form a seal around the surgical instrument.
30. The method of embodiment 29, further comprising providing a predetermined gas pressure within a space between the first and second sealing elements.

### BRIEF DESCRIPTION OF THE DRAWINGS

The invention will be more fully understood from the following detailed description taken in conjunction with the accompanying drawings, in which:

FIG. 1 is a perspective view of one embodiment of a surgical access device having a port with a non-resealable membrane disposed therein;

FIG. 2A is top view of one embodiment of a non-resealable membrane having puncture perforations formed therein;

FIG. 2B is a top view of another embodiment of a non-resealable membrane having puncture perforations formed therein;

FIG. 3A is a perspective view of one embodiment of a surgical access device having multiple sealing ports with non-resealable membranes;

FIG. 3B is a cross-sectional view of the surgical access device of FIG. 3A showing a surgical instrument disposed in one of the sealing ports;

FIG. 4 is an exploded view of an exemplary sealing port showing the various locations possible for non-resealable membranes;

FIG. 5 is a cross-sectional view of another exemplary sealing port showing various possible locations for non-resealable membranes;

FIG. 6 is a cross-sectional view of one embodiment of a surgical access device having sealing channels and non-resealable membranes disposed therein;

FIG. 7 is a cross-sectional view of another embodiment of a surgical access device having rotatable sealing ports therein showing various possible locations for non-resealable membranes;

FIG. 8 is a cross-sectional view of an exemplary recessed sealing port showing various possible locations for non-resealable membranes;

FIG. 9 is a cross-sectional view of an exemplary embodiment of a surgical access device having a hinged seal base showing various possible locations for non-resealable membranes within sealing ports;

FIG. 10 is a perspective view of one embodiment of a surgical access device having non-circular shaped sealing ports and non-circular shaped non-resealable membranes within the sealing ports;

FIG. 11 is a cross-sectional view of an exemplary surgical access device having sealing channels and non-resealable membranes disposed therein;

FIG. 12 is a cross-sectional view of another embodiment of a surgical access device having sealing ports in a deformable seal base showing various possible locations for non-resealable membranes;

FIG. 13A is a cross-sectional view of one embodiment of a surgical access device having a mechanically adjustable retractor and having a non-resealable membrane;

FIG. 13B is a cross-sectional view of the embodiment of FIG. 13A showing a retractor in an adjusted configuration;

FIG. 14A is a cross-sectional view of an exemplary surgical access device having a sealing element with a plurality of flexible membranes;

FIG. 14B is a top view of one of the plurality of flexible membranes of FIG. 14A;

FIG. 15 is an exploded view of an exemplary surgical access device with a non-resealable membrane and a support; and

FIG. 16 is a cross-sectional view of one embodiment of a surgical access device having two sealing elements separated by a pressurized gap filled with pellets.

### DETAILED DESCRIPTION OF THE INVENTION

Certain exemplary embodiments will now be described to provide an overall understanding of the principles of the structure, function, manufacture, and use of the devices and methods disclosed herein. One or more examples of these embodiments are illustrated in the accompanying drawings. Those of ordinary skill in the art will understand that the devices and methods specifically described herein and illustrated in the accompanying drawings are nonlimiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

The present invention generally provides improved methods and devices for accessing a body cavity. In certain exemplary embodiments, a surgical access device is provided and can include a housing having an access tube extending distally therefrom. The housing can have at least one port formed therein that defines a working channel in communication with one or more working channels of the access tube. At least one non-resealable membrane can extend across the working channel in at least one of the housing and the access tube to provide a seal across the working channel until a surgical instrument is punctured through the membrane. Once punctured, the membrane is non-resealable. The non-resealable membrane seals the working channel of the surgical access device before use thereof to maintain insufflation in a body cavity during a surgical procedure. The use of such a non-resealable membrane is an advantageous improvement over the traditional use of channel seals, such as zero-closure valves and duck bill valves, that seal the working channel of a device when no instrument is inserted therethrough. Channel seals tend to have a larger profile which requires surgical access devices to have a certain size to accommodate their vertical length. The height of the device can limit angular freedom of instruments inserted through the device. Moreover, in many procedures channel seals only find use before a surgical procedure to seal the working channel during insufflation and are not required subsequent to insertion of a surgical instrument into the working channel. Thus, through the use of lower profile non-resealable membranes to seal the working channel before a surgical instrument is inserted, surgical access devices can have a smaller and less unwieldy profile leading to a more comfortable and efficient surgical procedure.

As noted above, the various surgical access devices described herein can include an access tube extending from the housing. The access tube is generally positioned within an opening in a patient's body and is used for forming a pathway through tissue to provide a working channel for inserting instruments into a body cavity. The access tube can have various configurations and can be rigid, semi-rigid, or flexible as needed in a particular procedure. In certain exemplary embodiments, the access tube can be in the form of a cannula, one or more flexible sealing channels, and/or a retractor. The housing can include one or more ports formed therein, with each port defining a working channel extending through the housing and aligned with one or more working channels of the access tube. The ports can be in the form of an opening in the housing or it can include structure that is fixedly or movably disposed in the housing. Any and all of the surgical access devices described herein can also include various other features, such as one or more ventilation ports to allow evacuation of smoke during procedures that utilize cautery and/or one or more insufflation ports through which the surgeon can insufflate the abdomen to cause pneumoperitenium, as described for example in U.S. Patent Application No. 2006/0247673 entitled "Multi-port Laparoscopic Access Device" filed November 2, 2006 and incorporated herein by reference in its entirety. The insufflation port can be any size and can accept a leur lock or a needle, as will be appreciated by those skilled in the art.

In use, the surgical access devices disclosed herein can provide access to a patient's body cavity. The access tube can be positioned within an opening in tissue such that a distal portion of the access tube extends into a patient's body cavity and a proximal portion is coupled to the housing positioned adjacent to the patient's skin on an exterior of the patient's body. A lumen(s) in the access tube can form a pathway through the opening in tissue so that surgical instruments can be inserted from outside the body to an interior body cavity. The elasticity of the skin of the patient can assist in the retention of the access tube in the body opening or incision made in the body. The access tube can be placed in any opening within a patient's body, whether a natural orifice or an opening made by an incision. For example, the access tube can be placed through the umbilicus, endoscopically including, vaginally, percutaneously, etc. In one embodiment, the access tube can be substantially flexible so that it can easily be maneuvered into and within tissue as needed. The access tube can be formed of any suitable material known in the art, for example silicone, urethane, thermoplastic elastomer, and rubber.

Typically, during surgical procedures in a body cavity, such as the abdomen, insufflation is provided through the surgical access device to expand the body cavity to facilitate the surgical procedure. Thus, in order to maintain insufflation within the body cavity, most surgical access devices include at least one seal disposed therein to prevent air and/or gas from escaping when surgical instruments are inserted therethrough. Various sealing elements are known in the art, but typically the surgical access device can include at least one instrument seal that forms a seal around an instrument disposed therethrough, but otherwise does not form a seal when no instrument is disposed therethrough. One or more instrument seals can be disposed at various locations within the surgical access device. The instrument seal can be disposed within, for example, a port formed in the housing, across a body of the housing itself, and/or within or across a working channel access tube. In use, an instrument can be passed through a center opening of the instrument seal and the instrument seal can engage and form a seal around an outer surface of the instrument to thereby prevent the passage of fluids and gas through the seal. When no instrument is disposed therethrough, the center opening will generally not form a seal in the working channel. Exemplary instrument seal configurations are described in more detail in U.S. Publication No. 2004/0230161 entitled "Trocar Seal Assembly," filed on March 31, 2004, and U.S. Application Serial No. 10/687,502 entitled "Conical Trocar Seal," filed on October 15, 2003, which are hereby incorporated by reference in their entireties.

Typically, at least one channel seal that seals the working channel when no instrument is disposed therethrough would also be used in combination with the instrument seal. However, as noted above, channel seals tend to be larger in size and require the housing to be enlarged to accommodate their profile. During many surgical procedures, once insufflation of a body cavity is achieved and a surgical instrument is inserted into the working channel, the surgical instrument is not removed until completion of the surgical procedure. Thus, typical channel seals are only needed for initial sealing of the working channel for insufflation. Accordingly, the low profile non-resealable membrane of the present invention can be a useful alternative to traditional channel seals, however the non-resealable membrane can be used in combination with channel seals or with any other type of seal known in the art, including combination instrument and channel seals.

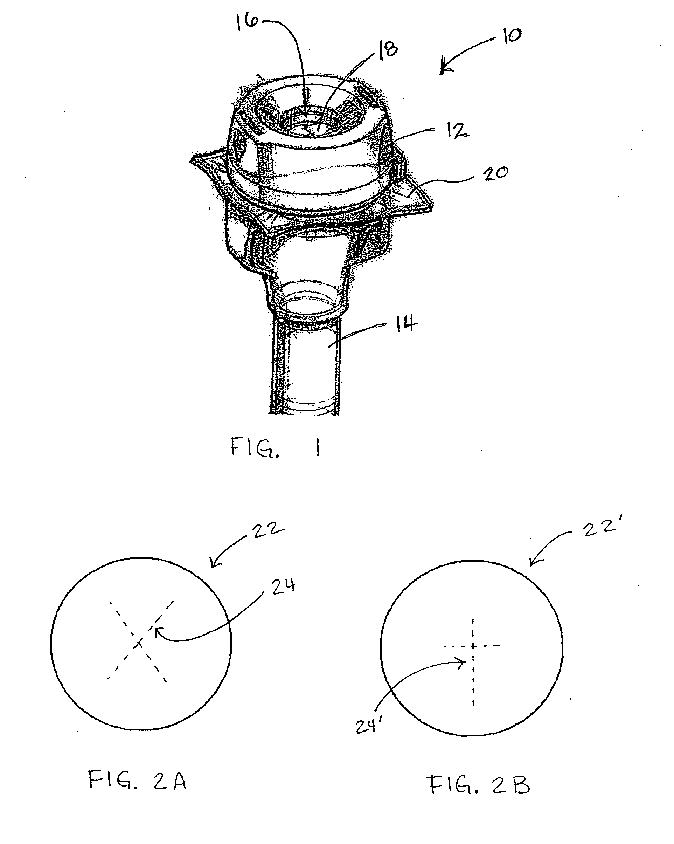

In one embodiment shown in FIG. 1, a surgical access device 10 is provided having a housing 12 with a cannula 14 extending therefrom. The housing 12 can include a port 16 defining a working channel extending through the housing 12 and the cannula 14. The port 16 can include a sealing element 18, for example, an instrument seal, disposed therein. The device 10 can also include a non-resealable membrane 20 extending across the working channel of the device 10. The non-resealable membrane 20 can generally be a substantially thin, flexible membrane extending substantially taught across the working channel of the device 10. In this way, the non-resealable membrane 20 seals the working channel of the device 10 before an instrument is inserted into the sealing port 16. Once the non-resealable membrane 20 is punctured by a surgical instrument, it will no longer be able to seal the working channel of the device 10. The sealing element 18 can form a seal around the surgical instrument, preventing escape of insufflation gas, at least until the instrument is removed.

The non-resealable membranes described herein can generally be formed of a flexible and/or resilient material, the flexibility and/or resiliency of which can vary depending on the application. The non-resealable membrane can generally be a relatively thin membrane that is able to seal a working channel of a surgical access device while remaining relatively easy to puncture by a surgical instrument. As will be appreciated, however, the non-resealable membrane can have any thickness as required and/or can vary in thickness over its area. In addition, the non-resealable membrane can have any desired tear or puncture strength. For example, the non-resealable membrane can be made to tear or puncture in response to insertion of a sharp surgical instrument and/or can be made to tear or puncture in response to insertion of a blunt surgical instrument. In either case, the non-resealable membrane can be configured to tear or puncture in response to varying degrees of force, from a small amount of force to a large amount of force, as needed. The non-resealable membrane can be formed of any suitable material known in the art, including, but not limited to polyurethane, silicone, cellophane.

In any of the embodiments disclosed herein, any of the non-resealable membranes described can generally be positioned anywhere within the housing and/or access tube. For example, the non-resealable membrane can be positioned above or below a sealing element in a sealing port, as well as adjacent thereto or spaced apart therefrom. The non-resealable membrane can also be positioned across any portion of the housing, and need not extend across the entire housing as shown in FIG. 1. Various techniques known in the art can be used to position the membrane within the housing or access tube such that it forms a fluid-tight seal across the working channel. For example, the membrane can be captured between two ring members or other structures that define the working channel. O-rings or other sealing techniques can optionally be used to prevent fluid leakage. In the embodiment shown in FIG. 1, the membrane is captured in a distal portion of the housing, and a lid, which contains the instrument seal, removably attaches to the distal portion of the housing. In other embodiments, the membrane can be captured between the lid and the distal portion of the housing. Such a configuration allows the surgical access device to be reused by removing the lid, replacing the membrane, and re-attaching the lid.

In addition, the non-resealable membrane can have any cross-sectional profile known in the art. For example, the non-resealable membrane can have a generally flat profile such that is extends laterally across a port or other working channel in a surgical access device orthogonal to a longitudinal axis of the port or at an angle greater than or less than 90 degrees with respect to a longitudinal axis of the sealing port. The non-resealable membrane can also have a preformed cross-sectional profile with a shape to match a particular component of a surgical access device. For example, the non-resealable membrane can have a conical profile to match a conical seal, or the non-resealable membrane can have a concave, hemispherical, or convex shape. The perimeter shape of the membrane can also vary, such as circular, square, rectangular, triangular, etc. A person skilled in the art will appreciate the variety of profiles that the non-resealable membrane can have. Embodiments of the non-resealable membrane having various positions and profiles within surgical access devices will be described below. Further details concerning some of the various embodiments described below that are not particularly relevant to the current invention can be found in U.S. Application No. 12/242,765 entitled "Surgical Access Device" filed on September 30, 2008; U.S. Application No. 12/242,711 entitled "Surgical Access Device with Protective Element" filed on September 30, 2008; U.S. Application No. 12/242,721 entitled "Multiple Port Surgical Access Device" filed on September 30, 2008; U.S. Application No. 12/242,726 entitled "Variable Surgical Access Device" filed on September 30, 2008, all of which are hereby incorporated by reference in their entireties.

As noted above, a surgical instrument can puncture the non-resealable membrane during insertion into the working channel of a surgical access device. Any and all of the non-resealable membrane embodiments described herein can thus have certain features to assist in the puncturing, as well as to direct a user to the correct puncture location. For example, as shown in FIGS. 2A and 2B, non-resealable membranes 22, 22' can have a plurality of perforations 24, 24' formed therein that provide an area of the non-resealable membranes 22, 22' that can be more easily punctured by a surgical instrument. As shown, the perforations 24, 24' can be small, for example, less than a millimeter in diameter or length, and can take the form of an "X," as shown in FIG. 2A, a "T," as shown in FIG. 2B, or any other shape preferred. In some embodiments, the perforations 24, 24' can be weakened portions of the membranes 22, 22', while in other embodiments, the perforations 24, 24' can extend entirely through the membranes 22, 22'. Even if the perforations 24, 24' extend entirely through the membranes 22, 22', the small size presents a negligible effect on maintaining insufflation in the working channel. In addition, the perforations 24, 24' could also simply be markings on the membranes 22, 22' to direct a user to the proper puncture location.

In any and all of the embodiments described herein, perforations formed in a particular non-resealable membrane can also be configured to divide the non-resealable membrane in such a way as to provide protection to a sealing element. For example, a non-resealable membrane can be placed proximally adjacent to a sealing element. A center point can be aligned with a central axis of the sealing element. When punctured, the non-resealable membrane can divide into flaps that provide a protective layer over the sealing element and protect it from puncture by a sharp surgical instrument. The non-resealable membrane can essentially function as a traditionally known seal protector, as will be appreciated by those skilled in the art.

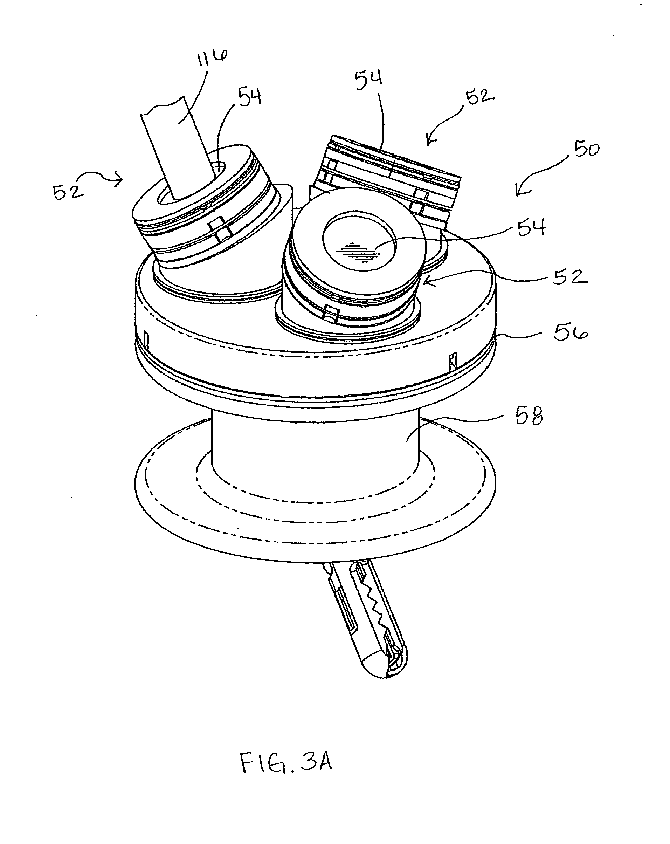

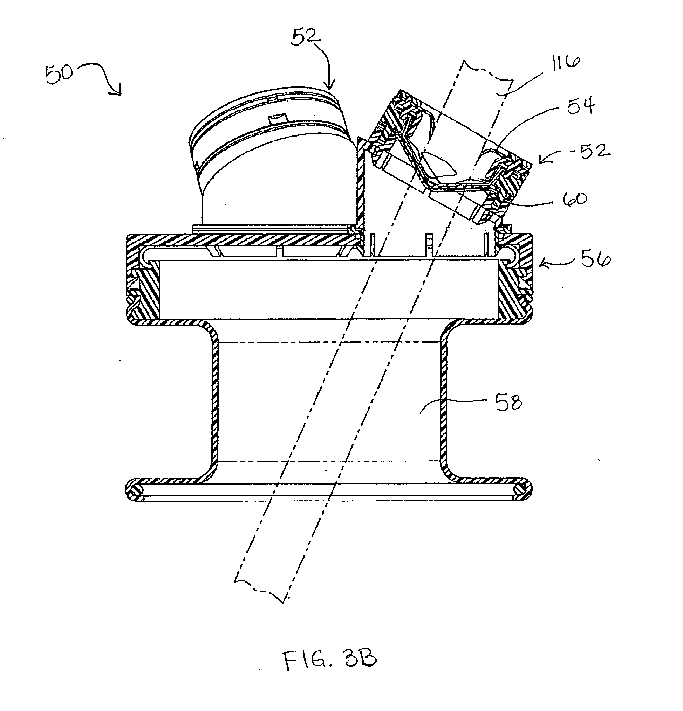

In one exemplary embodiment shown in FIGS. 3A and 3B, a surgical access device 50 is provided having a plurality of sealing ports 52 extending through a housing 56 at various angular orientations. The housing 56 can support the sealing ports 52 and a retractor 58 extending from the housing 56. While any number of sealing ports 52 can be formed in the housing 56, in the embodiment shown in FIGS. 3A-3B, three sealing ports 52 extend through the surgical access device 50. The sealing ports 52 can each have a non-resealable membrane 54 disposed across a top portion of the sealing ports 52 to seal the working channel when no surgical instrument is inserted therethrough. The sealing ports 52 can also each have sealing elements 60 disposed therein configured to form a seal around a surgical instrument inserted therethrough. In this way, the working channel of the device 50 is sealed by the non-resealable membrane to maintain insufflation until a surgical instrument 116 punctures the non-resealable membrane 54 during insertion into the sealing port 52. The seal element 60 then forms a seal around the surgical instrument 116 to maintain insufflation within the working channel.

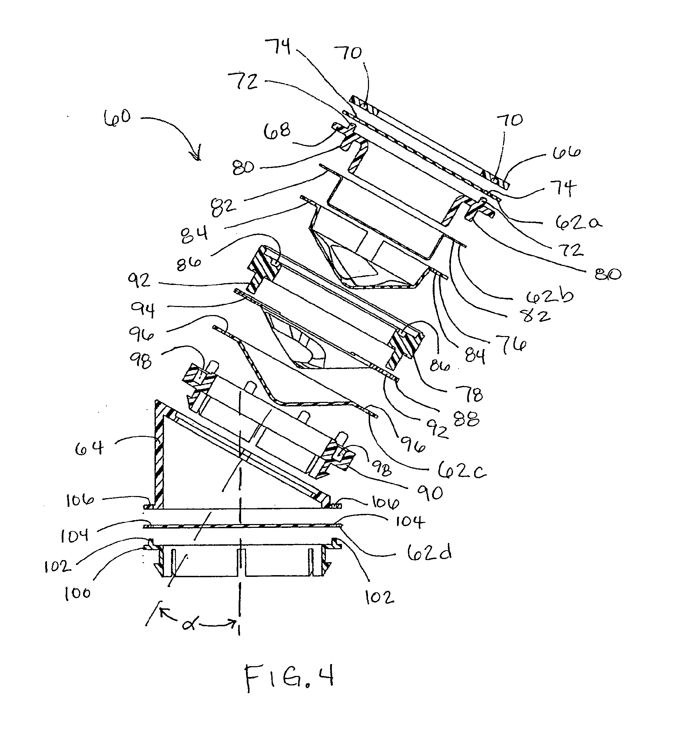

As noted above, a non-resealable membrane can be positioned any where within a sealing port, a housing, and/or an access tube of a surgical access device. FIG. 4 illustrates one embodiment of a sealing port 60 showing various possibilities for placement of a non-resealable membrane 62. The possible locations are designated as non-resealable membranes 62a, 62b, 62c, and 62d within the sealing port 60. A single non-resealable membrane 62 can be used at any one of the locations illustrated, or in some embodiments, a non-resealable membrane 62 can be used at two or more of the possible locations illustrated.

As shown, the sealing port 60 can generally include a port frame 64, in which the other sealing port components can be disposed. Beginning from the top-most portion of the sealing port 60, a non-resealable membrane 62a is shown positioned laterally across a top of the sealing port 60 such that a surgical instrument first punctures the non-resealable membrane 62 before entering the sealing port 60. Non-resealable membrane 62a is generally thin and flat and can be positioned orthogonally to a central longitudinal axis of the sealing port 60. The non-resealable membrane 62a can be secured in place by a clamp ring 66 and a clamp base 68. The clamp base 68 and the clamp ring 66 can be mated together with the sealing membrane 62a by any mechanism known in the art, including press fit, interference fit, screws, adhesives, etc. In one embodiment, the clamp base 68 can have mating protrusions 72 formed around an outer circumference thereof that extend proximally from a top surface of the clamp base 68 and extend through openings 74 formed around an outer circumference of the non-resealable membrane 62a and into indentations 70 formed around an outer circumference of the clamp ring 66. In this way, the non-resealable membrane 62a is held taught between the clamp ring 66 and the clamp base 68 to seal the working channel before a surgical instrument is inserted.

Another possible location for a non-resealable membrane 62 is illustrated by non-resealable membrane 62b. Non-resealable membrane 62b can be preformed into a particular shape having a cross-sectional profile that matches a portion of the sealing port 60. As shown, the non-resealable membrane 62b is preformed to match a shape of the clamp base 68 and thus has a cylindrical cup-like shape. The non-resealable membrane 62b can be positioned to extend around an outside of the clamp base 68 and to fit within a seal protector 76 and a sealing element frame 78. The membrane 62b can alternatively replace the seal protector 76 and sit directly within a seal 88. The clamp base 68, the seal protector 76, and the sealing element frame 78 can be mated together with the non-resealable membrane 62b by any mechanism known in the art, including press fit, interference fit, screws, adhesives, etc. In the illustrated embodiment, the clamp base 68 can have protrusions 80 formed around an outer circumference of a bottom surface thereof that can extend through openings 82 and 84 formed around an outer circumference of the non-resealable membrane 62b and the seal protector 76, respectively, and into indentations 86 formed around an outer circumference of the sealing element frame 78. In this way, the non-resealable membrane 62b fits the profile of various components in the sealing port 60 and forms a seal across the working channel before a surgical instrument is inserted therethrough.

Another possible location for the non-resealable membrane 62 is illustrated by non-resealable membrane 62c. Non-resealable membrane 62c can be preformed into a conical or triangular shape to match a profile of the seal 88. The non-resealable membrane 62c can be placed distally adjacent to the seal 88 and will thus be punctured after a surgical instrument is inserted through the seal 88. The non-resealable membrane 62c is generally secured between the sealing element frame 78 and the sealing element base 80 by any mechanism known in the art, including press fit, interference fit, screws, adhesives, etc. In the illustrated embodiment, protrusions 92 extending distally from an outer circumference of a bottom surface of the sealing frame 78 can extend through openings 94 and 96 formed in an outer circumference of the seal 88 and the non-resealable membrane 62c, respectively, and into indentations 98 formed in the sealing element base 80. In this way, the non-resealable membrane 62c fits the profile of the seal 88 to form a seal across the working channel before a surgical instrument is inserted therethrough.

Another possible location for the non-resealable membrane 62 is illustrated by non-resealable membrane 62d. Non-resealable membrane 62d can extend laterally across the working channel below the port frame 64 such that it is punctured after a surgical instrument is inserted through the sealing port 60. The non-resealable membrane 62d can have a central longitudinal axis disposed at an angle less than or greater than 90 degrees with respect to a central longitudinal axis of the sealing port 60. In the illustrated embodiment, the angle α is about 45 degrees. The non-resealable membrane 62d can generally be secured between the port frame 64 and the port base 100 by any mechanism known in the art, including press fit, interference fit, screws, adhesives, etc. In the illustrated embodiment, protrusions 102 extend proximally from an outer circumference of a top surface of the port base 100 and can extend through openings 104 formed around an outer circumference of the non-resealable membrane 62d and into openings 106 formed around an outer circumference of the port frame 64. In this way, the non-resealable membrane 62d seals the working channel of a surgical access device before a surgical instrument is inserted therethrough.

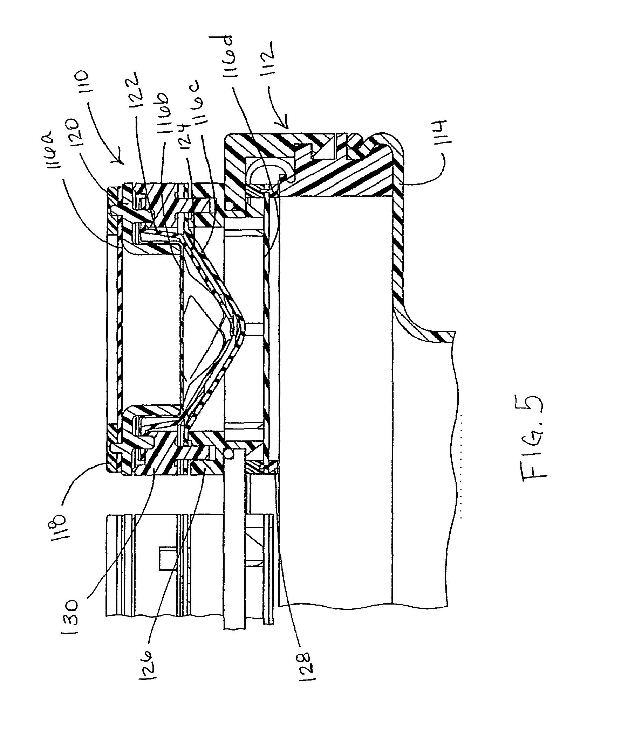

FIG. 5 illustrates another embodiment of a non-resealable membrane 116 within a sealing port 110 disposed within a housing 112 having a retractor 114 extending therefrom. This particular embodiment illustrates the various possible positions for the non-resealable membrane 116 within a sealing port 110 that is axially aligned, i.e., co-axial, with the retractor 114, as compared with the sealing port 60 of FIG. 4 which is axially offset or angled with respect to a longitudinal axis of the retractor. The various possible locations for the non-resealable membrane 116 are represented in FIG. 5 by non-resealable membranes 116a, 116b, 116c, and 116d. Similar to the embodiment shown in FIG. 4, non-resealable membrane 116a can be positioned between a clamp ring 118 and a clamp base 120. Non-resealable membrane 116b can be positioned between the clamp base 120 and a seal protector 122 such that it is proximally adjacent to the seal protector 122. Non-resealable membrane 116c can be positioned distally adjacent to a seal 124 between the seal 124 and a sealing element frame 130. Non-resealable membrane 116d can be positioned distally to the sealing port 110 between a port frame 126 and a port base 128. A person skilled in the art will appreciate that in some embodiments, non-resealable membrane 116c could be positioned proximally to the seal 124 in place of the seal protector 122 to thereby act as both a working channel seal before puncture and as a seal protector after puncture.

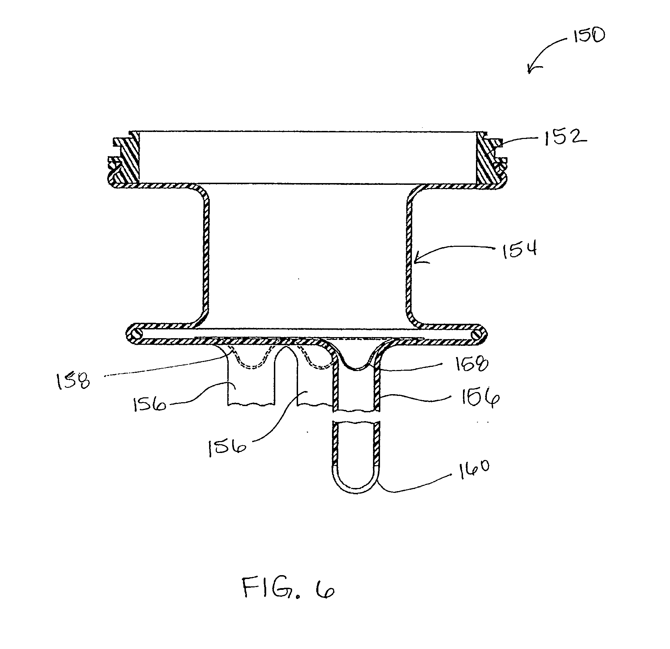

In another embodiment shown in FIG. 6, a surgical access device 150 is provided having a housing 152 with a retractor 154 extending therefrom. One or more flexible elongate seal channels 156 can extend distally from the retractor 154 to form a seal around an instrument inserted therein. A distal-most portion 160 of each sealing channel 156 can have sealing flaps that can form a seal around a surgical instrument inserted therethrough but do not necessarily (although they can) seal the working channel when no instrument is inserted therethrough. Non-resealable membranes 158 can be positioned near an entrance or opening into the seal channels 156. Each non-resealable membrane 158 can have a substantially conical or hemispherical shape to extend slightly into the opening of the seal channels 156 to thereby provide a one time seal of the channel 156. In some embodiments, the various non-resealable membranes 158 disposed in the entrances of the seal channels 156 can be connected together in a single flexible membrane across a base of the retractor 154. In other embodiments, the non-resealable membranes 158 can each be individually formed with their respective seal channel 156. As noted above, pre-formed puncture locations or other indications or markings can be made on the non-resealable membrane 158 to direct a user to the correct puncture location.

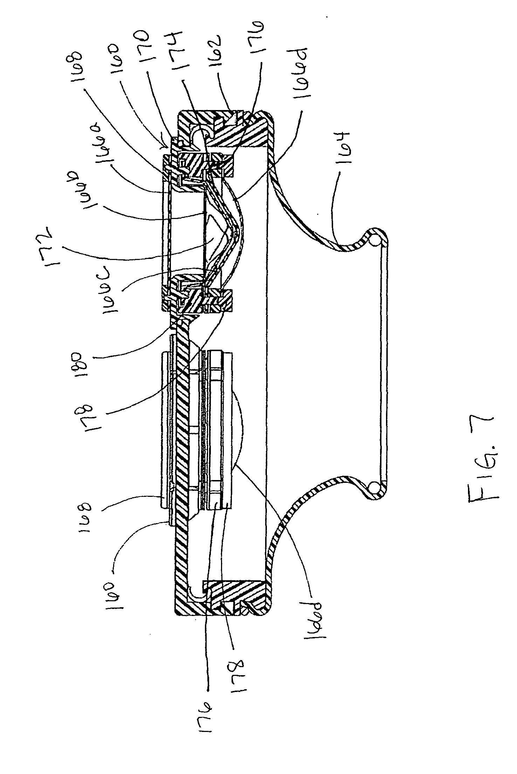

FIG. 7 illustrates another embodiment of a non-resealable membrane 166 disposed within a sealing port 160. A housing 162 is provided having a retractor 164 extending therefrom and having a plurality of sealing ports 160 extending therethrough. Each sealing port 160 can be rotatable relative to the housing 162. This embodiment illustrates the various possible positions for the non-resealable membrane 166 within the rotatable sealing ports 160. The various possible locations for the non-resealable membrane 166 are represented by non-resealable membranes 166a, 166b, 166c, and 166d. Similar to the embodiment shown in FIGS. 4 and 5, non-resealable membrane 166a can be positioned within the sealing port 160 between a clamp ring 168 and a clamp base 170. The non-resealable membrane 166b can be positioned between the clamp base 170 and a seal protector 172 such that it is proximally adjacent to the seal protector 172. Non-resealable membrane 166c can be positioned distally adjacent to a seal 174 between the seal 174 and a sealing element frame 180. Non-resealable membrane 166d can have a hemispherical shape so that it extends substantially below the seal 174 and can be positioned distally to the sealing port 160 between a port frame 176 and a port base 178.

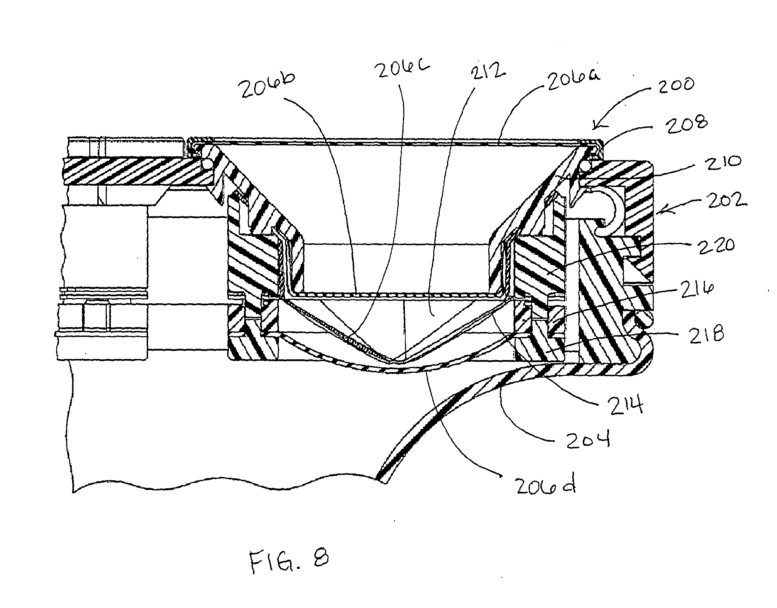

FIG. 8 illustrates another embodiment of a non-resealable membrane 206 within a sealing port 200 that can be disposed within a housing 202 having a retractor 204 extending therefrom. This embodiment illustrates the various possible positions for the non-resealable membrane 206 within a recessed sealing port 200. The various possible locations for the non-resealable membrane 206 are represented by non-resealable membranes 206a, 206b, 206c, and 206d. The non-resealable membrane 206a can be positioned within the recessed sealing port 200 between a clamp ring 208 and a recessed clamp base 210. The non-resealable membrane 166b can be positioned between the recessed clamp base 210 and a seal protector 212 such that it is proximally adjacent to the seal protector 212. The non-resealable membrane 166c can be positioned distally adjacent to a seal 214 between the seal 214 and a sealing element frame 220. The non-resealable membrane 166d can have a hemispherical shape so that it extends substantially below the seal 214 and can be positioned distally to the sealing port 200 between a port frame 216 and a port base 218.

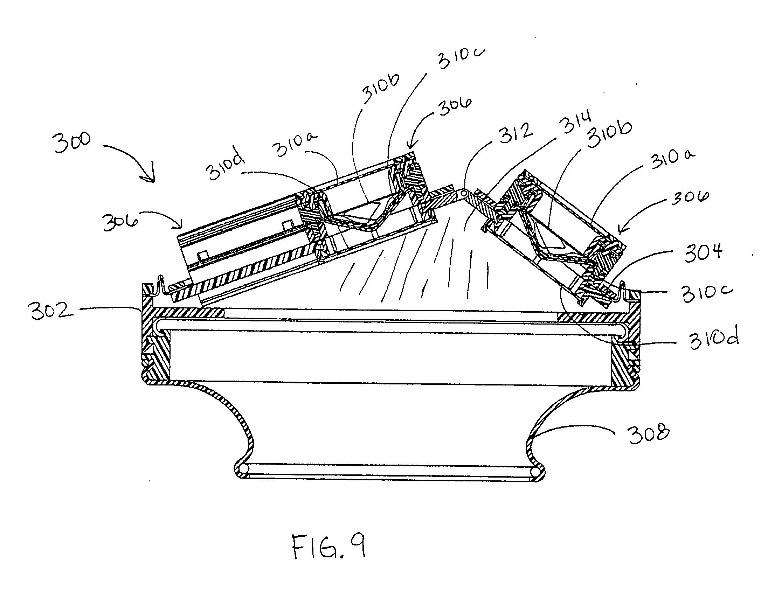

In still another embodiment shown in FIG. 9, a surgical access device 300 is provided having a housing 302 with a hinged seal base 304 and a retractor 308 extending from the housing 302. One or more sealing ports 306 can extend through the hinged seal base 304 and can be configured to receive surgical instruments therethrough. FIG. 9 illustrates various possible locations for a non-resealable member 310 within the sealing ports 306, represented as non-resealable members 310a, 310b, 310c, 310d in the same configuration as illustrated in the embodiment shown in detail in FIG. 5. The hinged seal base 304 can be selectively movable between various angular configurations relative to the housing 302 via a hinge 312 positioned within the seal base 304. A flexible membrane 314 can allow the seal base 304 to move relative to the housing 302 while maintaining insufflation. The non-resealable membranes 310a, 310b, 310c, 310d can seal the working channel of the device 300 before a surgical instrument is inserted therethrough.

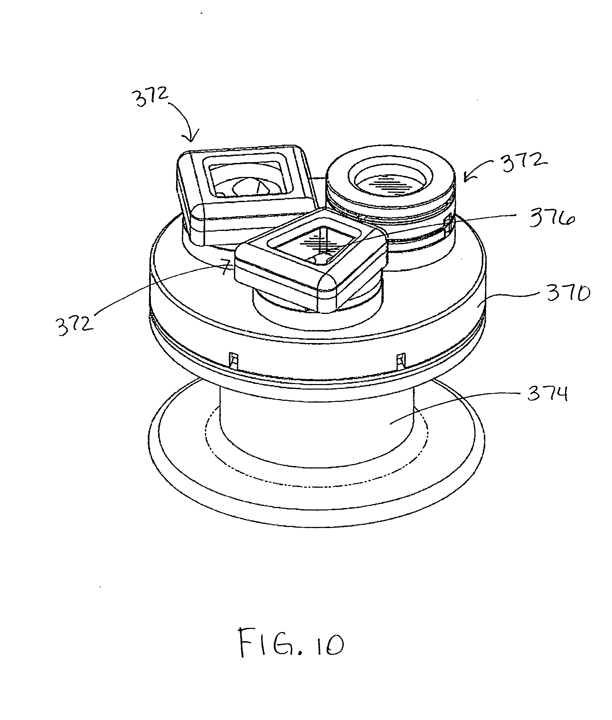

In one exemplary embodiment shown in FIG. 10, a housing 370 is provided having a plurality of sealing ports 372 extending therethrough and a retractor 374 extending therefrom. One or more of the sealing ports 372 can have a non-circular shaped opening to receive a surgical instrument having a non-circular cross-section. Thus, one or more non-resealable membranes, for example, non-resealable membrane 376, can have a non-circular shape to fit within the non-circular sealing port 372. The non-circular shaped sealing ports 372 and the non-resealable membranes 376 can have any shape known in the art, including but not limited to oval, triangular, quadrilaterals, polygons, etc.

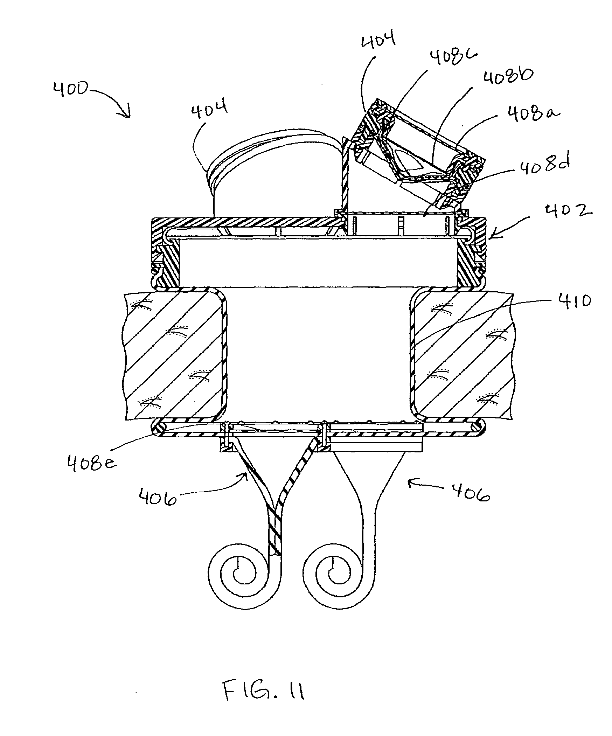

FIG. 11 illustrates one exemplary embodiment of a surgical access device 400 that generally includes a housing 402 having at least one sealing port 404 therein for receiving a surgical instrument therethrough. A retractor 410 can extend from the housing and can have at least one elongate flexible instrument channel 406 extending therefrom. The instrument channel 406 can be in communication with the sealing port 404 of the housing 402 to allow a surgical instrument to pass through the sealing port 404 and into the instrument channel 406. The instrument channel 406 can move from a natural resting state in a first collapsed configuration in which the instrument channel 406 is substantially sealed, as shown in FIG. 11, to a second expanded configuration upon insertion of a surgical instrument therethrough to form a seal around a surgical instrument. One or more non-resealable membranes 408 can be positioned at one or more various locations within the device 400. These various locations are represented by non-resealable membranes 408a, 408b, 408c, 408d, and 408e. The non-resealable membranes 408a, 408b, 408c, 408d can be positioned within the sealing port 404 in the same way as described above with respect to FIG. 4. The non-resealable membrane 408e can be positioned across an opening to the instrument channel 406. In this way, the non-resealable membrane 408e can seal the instrument channel 406 until a surgical instrument is inserted therethrough.

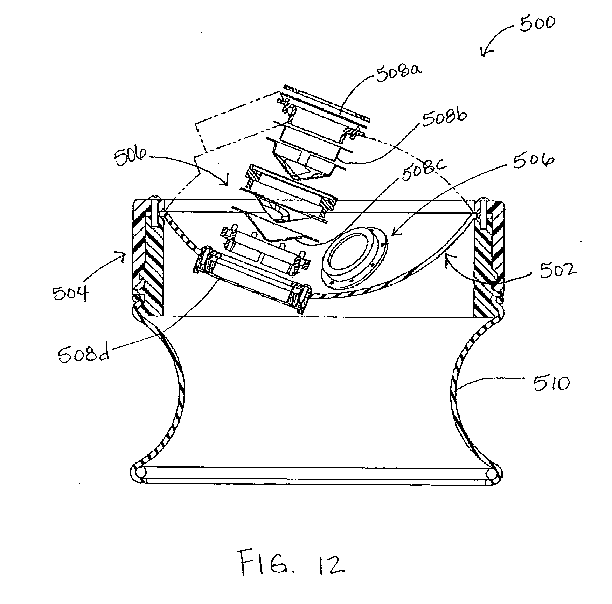

In another embodiment shown in FIG. 12, a surgical access device 500 is provided having a flexible seal base 502 and a housing 504 with a retractor 510 extending therefrom. The flexible seal base 502 can have one or more sealing ports 506 formed therethrough for receiving a surgical instrument. The sealing ports 506 can each have one or more non-resealable membranes 508, illustrated in their various locations by non-resealable membranes 508a, 508b, 508c, and 508d, positioned at one or more locations therein. The sealing ports 506 can have the same components as shown in FIGS. 4 and 5 and thus the non-resealable membranes 508a, 508b, 508c, 508d can be disposed within the sealing ports 506 in the same way. The flexible seal base 502 can have any shape, but in the illustrated embodiment is generally dome shaped. As a result, the flexible seal base 502 has a convex configuration in which it extends proximally from the housing 504, shown with a dotted line in FIG. 12, and a hemispherical configuration in which it extends distally into the housing 504, shown with a bold line. The flexible seal base 502 can be selectively moved between the convex and concave configurations as needed to reorient one or more of the sealing ports 506. Any one of the non-resealable membranes 508a, 508b, 508c, 508d can maintain insufflation within the device 500 until punctured by a surgical instrument.

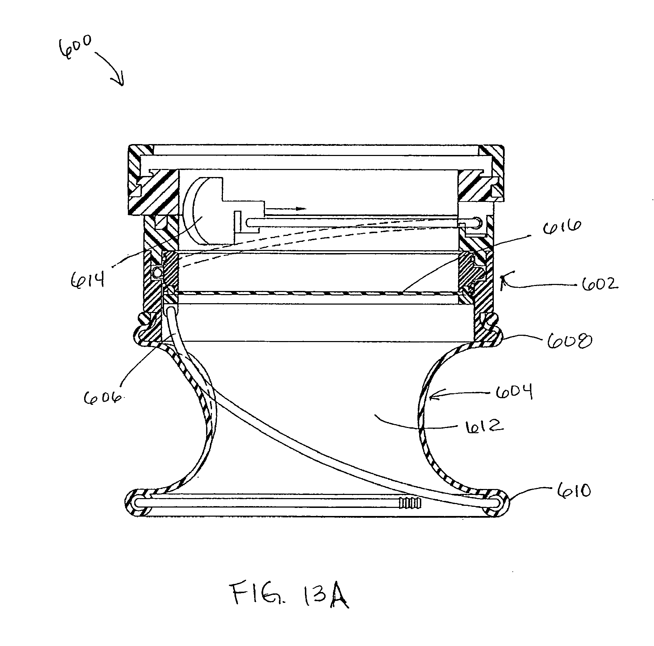

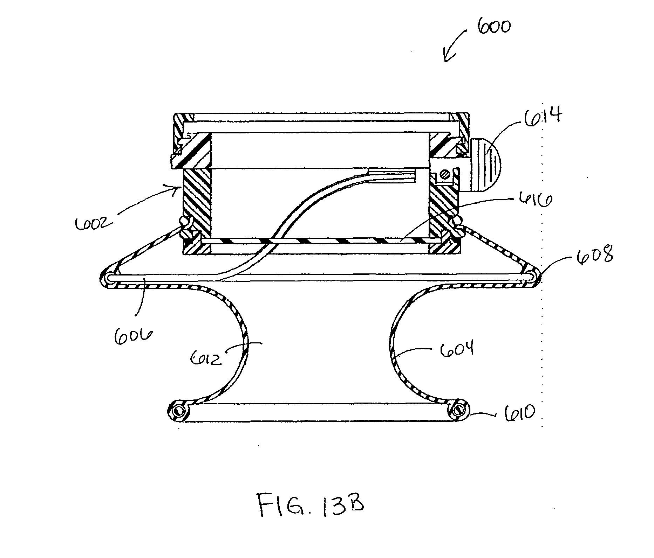

Another exemplary embodiment is illustrated in FIG. 13A and 13B. A surgical access device 600 is provided having a housing 602 with a retractor 604 extending therefrom. A non-resealable membrane 616 can be disposed across a working channel of the device 600 to seal the working channel before use. Once punctured, the non-resealable membrane 616 will no longer seal the working channel. Various mechanical or electrical mechanisms can also be associated with the device 600 to facilitate easier insertion and removal of the retractor 604 within an opening in a body. For example, in the illustrated embodiment, a tension wire 606, similar to a drawstring, is provided that allows a diameter of upper and lower retractor flanges 608, 610 to be changed for easier insertion into and removal from an opening in a body. The tension wire 606 terminates within the lower retractor flange 610 and extends at least part way therearound. The tension wire 606 then extends proximally around at least a portion of an elongate portion 612 of the retractor before extending around at least a portion of a circumference of the upper retractor flange 608. The tension wire 606 then extends through the housing 602, exits through one side and terminates in a finger tab 614.

The finger tab can be used to mechanically adjust the tension wire 606 by pushing and pulling the tension wire 606 along the above-described pathway to change the diameter of the upper and lower retractor flanges 608, 610. For example, in use, the retractor 604 can be moved from a position shown in FIG. 13A in which the retractor is generally in a resting or no tension configuration, to a position shown in FIG. 13B in which the tension wire 606 is under tension to increase the diameter of the upper retractor flange 608 and to decrease the diameter of the lower retractor flange 610. This allows the retractor 604 to be more easily positioned within an opening in a body. Once positioned within an opening, the tension wire 606 can be released to return to its relaxed configuration. For removal, the tension wire 606 can again be mechanically activated to change the diameter of the upper and lower retractor flanges 608, 610 to enable easier removal from an opening. More details regarding specific embodiments for adjusting a size of the retractor for ease of insertion and removal can be found in U.S. Application No. 12/420,146 entitled "Methods And Devices For Providing Access Into A Body Cavity," filed on even date herewith, which is incorporated by reference in its entirety.

In use, in any of the above-described embodiments, or any other embodiments described herein, a distal portion of a surgical access device can be inserted through a body cavity or an opening in tissue, whether natural or surgically formed, such that a working channel is created from outside the body, through tissue, to an interior surgical site. In some embodiments, a tension wire can be used to adjust a diameter of a lower ring on a retractor so that it can be more easily inserted into an opening in a body. Once inserted, the tension wire can be adjusted to allow the retractor ring to return to its normal configuration. An insufflation gas can be introduced through the surgical access device using an insufflation port or other mechanism known in the art to expand the interior surgical site. The one or more non-resealable membranes positioned in a sealing port, housing, and/or retractor, and extending across a working channel of the device, can provide a seal by preventing the insufflation gas from leaving the body cavity, thereby allowing insufflation to be achieved. It will be understood by a person skilled in the art that the insufflation port, while not illustrated in the above-described embodiments, can be positioned to introduce gas into the working channel at a location distal to the at least one membrane, assuming no other channel seals are provided. Such a configuration will allow the membrane to prevent the escape of gas from the body cavity through the working channel.

Once proper insufflation is achieved, a surgical instrument can be introduced into the device. The surgical instrument can be inserted through a sealing port, into the working channel of the device, and into an interior surgical site. In some embodiments, the non-resealable membrane extends across a top or proximal-most portion of the sealing port. In these embodiments, the surgical instrument will first puncture the non-resealable membrane and enter a sealing element within the sealing port that forms a seal around the surgical instrument. In other embodiments, the non-resealable membrane can be located at other positions within the sealing port. In such an embodiment, the surgical instrument will thus be inserted through a portion of the sealing port, and possibly through a sealing element, before puncturing the non-resealable membrane. In any of the embodiments, predefined puncture locations can guide a user to the correct location for puncturing the non-resealable membrane.

In one embodiment, the non-resealable membrane is positioned proximally adjacent to a sealing element so that when the non-resealable membrane is punctured, the non-resealable membrane divides into separate portions that lie against the sealing element and protect it against puncture or tear by the surgical instrument. In addition, once punctured, a larger non-resealable membrane extending across a top portion of the retractor can divide into separate portions and lie against an inside of the retractor to serve as a shield against tearing or puncturing thereof by the surgical instrument.

In general, once the non-resealable membrane is punctured, it will no longer provide a seal between the interior surgical site and the outside of the surgical access device. Accordingly, one or more sealing elements can form a seal around the surgical instrument in order to maintain insufflation within a body cavity. Once a surgical procedure is complete, the surgical instrument and the surgical access device can be removed from the body cavity. In some embodiments, a tension wire in communication with a retractor can be adjusted such that a lower retractor ring can be made smaller in diameter for easier removal from an opening in a patient's body.

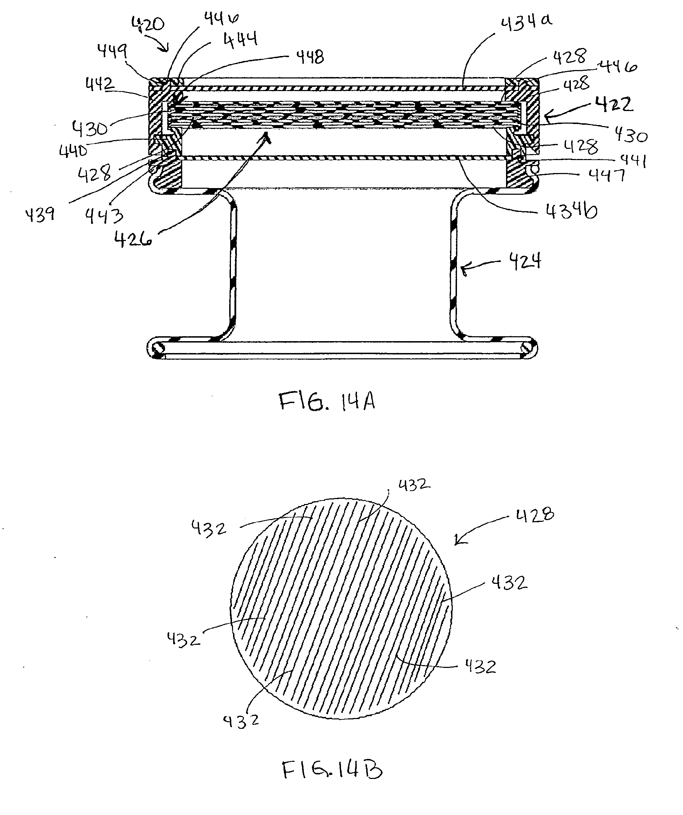

Another embodiment of a surgical access device is shown in FIG. 14A. A surgical access device 420 is provided having a housing 422 with a retractor 424 extending therefrom. A sealing element 426 can be positioned across a working channel of the housing 422 and the retractor 424 and it can function as an instrument seal and optionally as a channel seal. The sealing element 426 can be composed of a plurality of thin, flexible sealing membranes 428 positioned one on top of another within a frame 430. Each sealing membrane 428 can have a plurality of slits 432 formed therethrough positioned parallel to one another in the sealing membrane 428, shown most clearly in FIG. 14B. The number of slits in each sealing membrane 428 can vary but in one exemplary embodiment can be between about one and about twenty. In one embodiment, there can be fifteen slits in each membrane. The membranes can also have varying numbers of slits as needed.

The slits 432 can flex open and closed to receive a surgical instrument therethrough. When stacked one on top of another, the slits 432 in each sealing membrane 428 can be oriented such that they are unaligned or offset (e.g. non-parallel) with respect to the slits 432 in the other sealing membranes 428. In this way, when a surgical instrument is inserted through the sealing element 426, the overlapping slits 432 can flex open to receive the instrument and flex closed to from a seal around the instrument. Depending on the number of sealing membranes 428 used in a particular sealing element 426, the plurality of slits 432 can overlap each other such that some or all space around the surgical instrument is sealed. In some embodiments, the sealing element 426 can form a seal around a surgical instrument 360 degrees therearound. There can be any number of sealing membranes 428 included in the sealing element 426, including between one and twenty. In some particular embodiments, the number of sealing membranes 428 can be five, and in other embodiments, the number of sealing membranes 428 can be ten. As will be appreciated by those skilled in the art, the sealing element 426 can simultaneously form a seal around any number of surgical instruments inserted therethrough as space within the housing 422 will allow.

As noted above, the sealing membranes 428 can be held together within the frame 430, and the sealing element 426 can be secured within the housing 422 using any technique, such as between a seal base 440 and a clamp ring base 442. Any mechanism known in the art can be used to secured the sealing element 426 between the seal base 440 and the clamp ring base 442 including, but not limited to, adhesives, screws, notches, pins, press fit, and/or interference fit.

In addition to the sealing element 426, the device 420 can also optionally include one or more non-resealable membranes 434, designated in their possible locations by non-resealable membranes 434a, 434b. As shown in FIG. 14A, the non-resealable membrane 434a can be positioned proximally to the sealing element 426 and thus can seal a working channel of the device 420 before an instrument is inserted therein. Alternatively, or in addition, the non-resealable membrane 434b can be positioned distally to the sealing element 426 and a surgical instrument inserted into the working channel of the device 420 will have a seal formed therearound by the sealing element 426 before puncturing the non-resealable membrane 434b.

The non-resealable membrane 434a can be secured within the housing 422 using any technique, such as between a clamp ring 444 and the clamp ring base 442. While the clamp ring 444 and the clamp ring base 442 can secure the non-resealable membrane 434a therebetween using any mechanism known in the art, in the illustrated embodiment, the clamp ring base 442 can have mating protrusions 446 formed around an outer circumference thereof and extending proximally therefrom. The mating protrusions 446 can be configured to extend through openings 448 formed around an outer circumference of the non-resealable membrane 434a and into openings 449 formed around an outer circumference of the clamp ring 442. Likewise, the non-resealable membrane 434b can be secured within the housing 422 between the seal base 440 and a housing seat 447. The housing seat 447 can have mating protrusions 443 formed around an outer circumference thereof extending proximally therefrom configured to extend through openings 441 formed around an outer circumference of the non-resealable membrane 434b and into openings 439 formed around an outer circumference of the seal base 440.

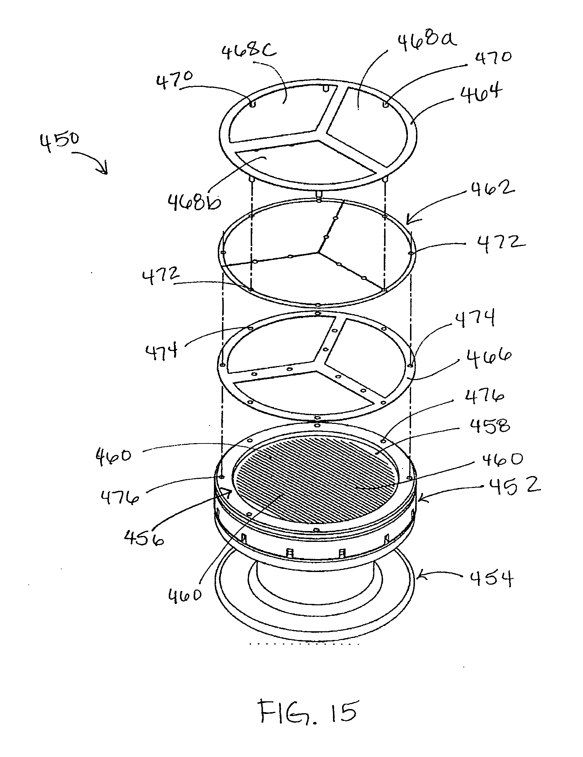

In another exemplary embodiment shown in FIG. 15, a surgical access device 450 is provided having a housing 452 with a retractor 454 extending therefrom. A sealing element 456 having a plurality of flexible membranes 458 can be disposed across a working channel of the device 450. Each of the plurality of membranes 458 can have a plurality of parallel slits 460 formed therein that can form a seal around a surgical instrument as described above with respect to FIGS. 14A and 14B, and the membranes can be offset from one another such that the slits are non-parallel. One or more non-resealable membranes 462 can be positioned within the housing 452 and can be positioned proximally and/or distally to the sealing element 456.

In the embodiment shown in FIG. 15, the non-resealable membrane 462 is positioned proximally to the sealing element 456 and can be secured within the housing between upper and lower frames 464, 466. The upper and lower frames 464, 466 can act to hold the non-resealable membrane 462 relatively taught for ease of puncturing, and it can be divided into one or more sections, for example, three sections 468a, 468b, 468c, for one or more surgical instruments. The non-resealable membrane 462 can be secured between the upper and lower frames 464, 466 by any method known in the art, for example, screws, adhesives, press fit, etc. In the illustrated embodiment, the upper frame 464 has a plurality of mating protrusions 470 formed around an outer circumference thereof that can extend through openings 472 formed through an outer circumference of the non-resealable membrane 462, through openings 474 formed through an outer circumference of the lower frame 466, and into openings 476 formed around an outer circumference of the sealing element 456. In this way, the non-resealable membrane 462 is secured within the housing 452.

In use in the embodiment shown in FIG. 15, at least three surgical instrument can be inserted into the device 450. One surgical instrument can be inserted through each of the sections 468a, 468b, 468c. Each surgical instrument can puncture a particular section 468a, 468b, 468c of the non-resealable membrane 462 and can be inserted into the sealing element 456. As will be appreciated, the upper and lower frames 464, 466 allow one of the sections 468a, 468b, 468c to be punctured while the other sections remain under tension for puncturing by other surgical instruments. A person skilled in the art will appreciate that, while three sections are shown, the frame can have any shape and can define any number of sections.

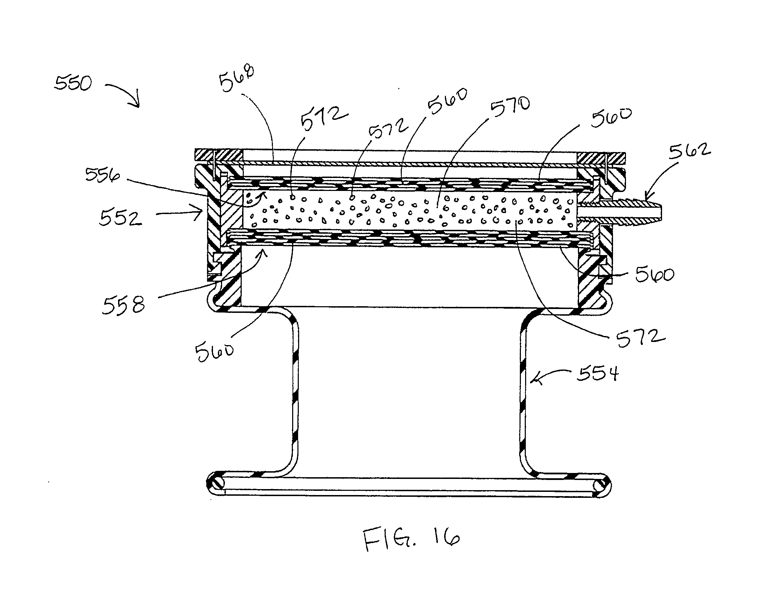

Another exemplary embodiment is shown in FIG. 16. A surgical access device 550 is provided having a housing 552 with a retractor 554 extending therefrom. One or more sealing elements and one or more non-resealable membranes can be positioned within the housing 552. In the illustrated embodiment, two sealing elements 556, 558 are positioned across a working channel of the device 550 within the housing 552 with a gap 570 therebetween. The sealing elements 556, 558 can each have a plurality of flexible membranes 560 stacked one on top of the other in a configuration similar to that described above with respect to FIGS. 14A and 14B. A plurality of parallel slits in each membrane 560 can be oriented such that the membranes 560 collectively form a seal around a surgical instrument inserted therethrough. In the illustrated embodiment, each sealing element 556, 558 can have about five flexible membranes 560, although they can contain as many or as few as needed in a particular application. The sealing elements 556, 558 can be secured between components of the housing in a way similar to that described above with respect to FIG. 14A.

The gap 570 between the sealing elements 556, 558 can have many configurations, and in some embodiments, the gap 570 can be filled with insufflation gas, as will be described in more detail below, and form a curtain of insufflation gas between the insufflation gas in the body and the sealing element 556 to help prevent leaks of insufflation gas from the body and through the sealing element 556. In other embodiments, the gap 570 can contain any material having discrete components, beads, or pellets 572. The pellets 572 can be small pieces of Styrofoam or PVC pellets, similar to a bean-bag filler material, and can be less dense than the flexible membranes 560. This configuration can allow for easier insertion of a surgical instrument through the sealing elements 556, 558 as there are fewer flexible membranes 560 that a surgical instrument must pass through at once, when compared with the embodiment in FIG. 14A. This is particularly advantageous for surgical instruments having a large diameter. In addition, the pellets 572 provide an area that can assist in maintaining insufflation below the sealing element 558, as it is difficult for insufflation gases to pass through the pellets 572 to leave the device 550. For example, if a leak forms in the sealing element 558, the pellets 572 will tend to move upward and press against the sealing element 556, thereby blocking the leak. The pellets 572 can be formed of any suitable material known in the art, including, but not limited to, polypropylene, polyethylene, polystyrene, and/or any other closed cell foamed rubber.

An insufflation port 562 can extend through a sidewall of the housing 552 and can be in communication with the gap 570 to provide a channel through which a predetermined pressure within the gap 570 can be maintained. In some embodiments, the insufflation port 562 can have separate vents to deliver insufflation gas to a body cavity through a working channel of the device while also delivering a predetermined pressure to the gap 570. The insufflation port 562 can deliver a predetermined pressure within the gap 570 between the sealing elements 556, 558 to assist in controlling the pressure of the insufflation gas curtain when no pellets 572 are disposed therein, and/or to assist in controlling movement of the pellets 572 within the gap 570 when pellets 572 are disposed therein. For example, when a surgical instrument is being removed from the device 550, the insufflation port 562 can provide a vacuum or negative pressure within the gap 570 that can prevent the pellets 572 from falling into a body cavity. The insufflation port 562 can also be used to provide a positive pressure within the gap 560 to cause the pellets to move to block any leaks that form.

As shown, the device 550 can also include a non-resealable membrane 568 extending across the working channel of the device. The non-resealable membrane 568 can have a single opening, or it can be divided into two or more sections in a manner as described above with respect to FIG. 15. In this way, the non-resealable membrane 568 seals the working channel of the device 550 while insufflation is achieved. In use, a surgical instrument can puncture the non-resealable membrane 568 before being inserted through the sealing elements 556, 558 and into a body cavity.

In use, for the embodiments described in FIGS. 14A-16, as well as any other embodiments described herein, a distal portion of a surgical access device can be inserted through a body cavity or an opening in tissue, whether natural or surgically formed, such that a working channel is created from outside the body, through tissue, to an interior surgical site. An insufflation gas can then be introduced through the surgical access device using an insufflation port or other mechanism known in the art to expand the interior surgical site. The one or more non-resealable membranes positioned in a sealing port, housing, and/or retractor, and extending across a working channel of the device, can provide a seal by preventing the insufflation gas from leaving the body cavity, thereby allowing insufflation to be achieved.

Once proper insufflation is achieved, a surgical instrument can be introduced into the device. The surgical instrument can be inserted through a sealing element having a plurality of flexible sealing membranes. Each flexible sealing membrane can have a plurality of parallel slits formed therein such that the surgical instrument is inserted through one of the slits in each flexible membrane. Due to the orientation of the flexible membranes, the slits overlap one another to form a seal around an entire circumference of the surgical instrument when it is disposed through the sealing element. One or more non-resealable membranes can be disposed in the housing and can be positioned above or below the sealing element. The surgical instrument can thus puncture the non-resealable membrane either before or after entering the sealing element. The non-resealable membrane will no longer seal the working channel once punctured, and the sealing element will provide a seal around the surgical instrument.

In some embodiments, the sealing element has two or more sections of flexible membranes with a gap filled with, for example, pellets, therebetween, such as the embodiment described above with respect to FIG. 16. In this case, the surgical instrument can be inserted through the top portion of the sealing element to extend through the gap filled with pellets and through the bottom portion of the flexible membrane. The gap between the two sections of flexible membranes can be maintained at a predetermined pressure to ensure that the pellets within the gap do not fall into a body cavity upon insertion or removal of a surgical instrument therein. While a surgical instrument is inserted within the sealing element, the pellets within the gap can act to prevent leaks from occurring by moving to block any leaks within the flexible membranes from escaping. As the surgical instrument is withdrawn from the device, a negative pressure can be applied to the gap between the sealing element sections to prevent any pellets from falling into a body cavity.

As will be appreciated by those skilled in the art, any and all of the housing, seal base, sealing port, non-resealable membrane, and access tube embodiments disclosed herein can be interchangeable with one another as needed. For example, a kit could include multiple housings, seal bases, sealing ports, and non-resealable membranes, with one or more access tubes. A release mechanism can be used to releasably attach the various housings, sealing ports, and non-resealable membranes to a access tube.

As surgical instruments are inserted through the surgical access device embodiments described herein, a risk can exist that a particularly sharp instrument may tear or puncture a portion of the access tube or nearby tissue. Accordingly, in any and all of the embodiments described herein, a safety shield can optionally be included to reduce the risk of tearing or puncture by a surgical instrument. In general the shield can be of a material that is relatively smooth to allow ease of passage of instruments, but resistant to tearing and puncture. For example, the shield can formed of silicone, urethane, thermoplastic elastomer, rubber, polyolefins, polyesters, nylons, fluoropolymers, and any other suitable materials known in the art. The shield can generally provide a liner for an access tube or tissue and can be detachable from a surgical access device so it can be used as needed in a particular procedure.

In any and all of the surgical access device embodiments disclosed herein, an engagement and/or release mechanism can be included to allow a housing to be separated from an access tube, to allow one portion of a housing to be separated from another portion of a housing, and/or to allow a sealing port to be separated from a housing. The engagement or release mechanism can be a latch, switch, c-clamp, tabs, push button, or any other mechanism known in the art that can be configured to release one portion of a device from another.

There are various features that can optionally be included with any and all of the surgical access device embodiments disclosed herein. For example, a component of the device, such as a housing, access tube, sealing port, etc., can have one or more lights formed thereon or around a circumference thereof to enable better visualization when inserted within a patient. As will be appreciated, any wavelength of light can be used for various applications, whether visible or invisible. Any number of ports can also be included on and/or through the surgical access devices to enable the use of various surgical techniques and devices as needed in a particular procedure. For example, openings and ports can allow for the introduction of pressurized gases, vacuum systems, energy sources such as radiofrequency and ultrasound, irrigation, imaging, etc. As will be appreciated by those skilled in the art, any of these techniques and devices can be removably attachable to the surgical access device and can be exchanged and manipulated as needed.

The devices disclosed herein can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces, and subsequent reassembly. In particular, the device can be disassembled, and any number of the particular pieces or parts of the device can be selectively replaced or removed in any combination. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

Preferably, the invention described herein will be processed before surgery. First, a new or used instrument is obtained and if necessary cleaned. The instrument can then be sterilized. In one sterilization technique, the instrument is placed in a closed and sealed container, such as a plastic or TYVEK bag. The container and instrument are then placed in a field of radiation that can penetrate the container, such as gamma radiation, x-rays, or high-energy electrons. The radiation kills bacteria on the instrument and in the container. The sterilized instrument can then be stored in the sterile container. The sealed container keeps the instrument sterile until it is opened in the medical facility.

It is preferred that device is sterilized. This can be done by any number of ways known to those skilled in the art including beta or gamma radiation, ethylene oxide, steam, and a liquid bath (e.g., cold soak).

One skilled in the art will appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.

## Claims

1. A surgical access device, comprising:
a housing having an access tube extending distally therefrom, the housing having at least one port formed therein and defining a working channel in communication with a working channel of the access tube; and
a non-resealable membrane extending across the working channel in at least one of the at least one port and the access tube, the membrane providing a seal across the working channel until a surgical instrument is punctured through the membrane.

2. The surgical access device of claim 1, wherein the at least one port includes at least one sealing element disposed therein configured to form a seal around an instrument inserted therethrough.

3. The surgical access device of claim 2, wherein the non-resealable membrane is positioned proximal to the at least one sealing element.

4. The surgical access device of claim 2, wherein the non-resealable membrane is positioned distal to the at least one sealing element.

5. The surgical access device of claim 1, wherein the non-resealable membrane includes a plurality of perforations formed therein configured to provide a predefined puncture location to facilitate puncturing of the membrane by a surgical instrument.

6. The surgical access device of claim 1, wherein the non-resealable membrane is substantially flat and extends across the at least one port in a direction perpendicular to a central longitudinal axis of the at least one port.

7. The surgical access device of claim 2, wherein the non-resealable membrane is positioned proximal to the at least one sealing element and is configured to protect the sealing element when a surgical instrument is inserted therethrough.

8. The surgical access device of claim 1, wherein the non-resealable membrane is positioned at an angle less than 90 degrees with respect to a central longitudinal axis of the port.

9. The surgical access device of claim 1, wherein the access tube includes at least one flexible elongate sealing element extending distally therefrom and configured to form a seal around a surgical instrument inserted therethrough.

10. The surgical access device of claim 1, wherein the access tube comprises a flexible retractor.

11. The surgical access device of claim 1, wherein the access tube comprises a rigid cannula.

12. The surgical access device of claim 1, wherein the non-resealable membrane includes a support member that is configured to provide support to at least one predefined region of the non-resealable membrane when a tool is inserted through the predefined region.

13. The surgical access device of claim 1, wherein the housing includes a plurality of ports, each port having a non-resealable membrane extending across a working channel of the port.

14. A method of providing surgical access within a body, comprising:
inserting a distal portion of a surgical access device through an opening in tissue to position a distal end of the surgical access device in a body cavity;
providing an insufflation gas through a working channel of the surgical access device to insufflate the body cavity, a membrane extending across the working channel forming a seal in the working channel that prevents escape of the insufflation gas from the body cavity; and
inserting a surgical instrument through the working channel of the surgical access device, the instrument puncturing the membrane such that the membrane is incapable of sealing the working channel when the surgical instrument is removed.

15. A surgical access device, comprising:
an access tube having an opening extending therethrough for forming a pathway through tissue into a body cavity; and
a housing coupled to the access tube and having at least one sealing member therein, the at least one sealing member having a plurality of sealing membranes positioned one on top of another with each sealing membrane having a plurality of slits formed therethrough, the plurality of sealing membranes being oriented such that the plurality of slits in each sealing membrane are substantially offset from one another to form a seal around a surgical instrument inserted through the at least one sealing member.
